Europäisches Patentamt

European Patent Office

Office européen des brevets

(11) Publication number: **0 141 304**
**B1**

(12) **EUROPEAN PATENT SPECIFICATION**

(45) Date of publication of patent specification: **01.02.89**

(21) Application number: **84112071.0**

(22) Date of filing: **09.10.84**

(51) Int. Cl.⁴: **C 12 N 15/00**

(54) **Method for the cell fusion of laver.**

(30) Priority: **18.10.83 JP 194726/83**
**31.05.84 JP 109667/84**

(43) Date of publication of application:
**15.05.85 Bulletin 85/20**

(45) Publication of the grant of the patent:
**01.02.89 Bulletin 89/05**

(84) Designated Contracting States:
**AT BE CH DE FR GB IT LI NL SE**

(56) References cited:
**NL-A-7 101 939**

**CHEMICAL ABSTRACTS, vol. 86, no. 7, 14th
February 1977, page 226, no. 39777j,
Columbus, Ohio, US; J.D.CLEMENT-METRAL:
"Preparation and some properties of
protoplasts from the red alga Porphyridium
cruentum" & J. MICROSC. BIOL. CELL. 1976,
26(2-3), 167-72**

(73) Proprietor: **KOASA TRADING CO.,LTD.**
**1-7 Meieki 5-chome Nakamura-Ku
Nagoya Aichi-Ken (JP)**

(72) Inventor: **Shibata, Teruhiko**
**22-15, Karatodai 4-chome Kita-ku
Kohbe Hyogo-ken (JP)**

(74) Representative: **Weisert, Annekäte, Dipl.-Ing.
Dr.-Ing.
Patentanwälte Kraus Weisert & Partner Thomas-
Wimmer-Ring 15
D-8000 München 22 (DE)**

Courier Press, Leamington Spa, England.

## Description

### Background of the invention
### 1) Field of the invention

This invention relates to a method for the cell fusion of laver by using monospores, carpospores or conchospores produced in the life cycle of laver.

### Description of the prior art

In recent years, extensive research and development concerning cell fusion as a genetic engineering technique have been being carried on and, with terrestrial plants, the stage of practical application has already been attained in some instances.

With marine algae (including red lavers), however, no successful case of cell fusion has been reported as yet.

This situation arises from the fact that, with terrestrial plants, protoplasts can be readily formed by using a commercially available enzyme preparation (e.g., Cellulase-Onozuka, Pectolyase or Macerozyme), while with marine algae and especially with red lavers, the polysaccharides constituting the skeletal components of their tissue resist treatment with such an enzyme preparation and thereby hinder the formation of sound protoplasts.

More specifically, the polysaccharides constituting the tissue of red lavers include xylan, mannan and porphyran. In particular, the cell wall consists of xylan formed into strong fibers and, therefore, cannot be hydrolyzed by an enzyme preparation as described above. Up to the present, there has been no report on an enzyme preparation having the ability to hydrolyze the aforesaid three polysaccharides at the same time.

In view of the above-described existing state of the art in which it is very difficult to prepare sound protoplasts by treating the tissue of laver with an enzyme, the present inventor has found that the cell fusion of laver can be achieved by paying attention to monospores, carpospores or conchospores produced in the life cycle of layer and applying conventional cell fusion techniques to those reproductive cells with the aid of a fusion inducing substance.

### Summary of the invention

The present invention has been made on the basis of the above-described finding and has enabled the cell fusion of laver without using protoplasts of laver.

Accordingly, it is the primary object of the present invention to provide a method for the cell fusion of laver not by using protoplasts of laver, but by using reproductive cells selected from the group consisting of monospores, carpospores and conchospores produced in the life cycle of laver.

Other objects of the present invention will be apparent from the following description.

### Detailed description of the invention

The characteristic feature of the present invention lies in the fact that monospores, carpospores or conchospores produced in the life cycle of laver are subjected to cell fusion with the aid of a fusion inducing substance.

Monospores for use in the cell fusion method of the present invention are spores produced from thalli of laver in the life cycle thereof. They become attached to laver netting, germinate to produce a rhizoid and form a cell wall, and then grow by cell division to produce thalli of laver. In this life cycle, monospores in the stage preceding the formation of a cell wall are gelatinous, amoeboid cells having a diameter of the order of 10—13 microns. They are considered to be in substantially the same form as protoplasts obtained by removing the cell wall from thalli of laver.

Carpospores are also spores produced from thalli of laver in the life cycle thereof. They part from the thalli of laver, become attached to shells or the like, and eventually penetrate thereinto to produce filaments known as the *Conchocelis* phase. They have a thin cell wall and, similarly to the aforesaid monospores, are considered to be in substantially the same form as protoplasts prepared from laver.

Furthermore, conchospores for use in the cell fusion method of the present invention are spores produced from the *Conchocelis* phase of laver in the life cycle thereof. They become attached to laver netting, germinate to produce a rhizoid and form a cell wall, and then grow by cell division to produce thalli of laver. Conchospores in the stage preceding the formation of a cell wall are gelatinous, amoeboid cells having a diameter of the order of 10—13 microns. They are considered to be in substantially the same form as protoplasts obtained by removing the cell wall from thalli of laver.

These reproductive cells can be artificially obtained as follows: In the case of monospores, young thalli of *Porphyra tenera* having a size of 0.2—1.0 mm or thalli of *Porphyra yezoensis* having a size of 1.0—10 cm are cultured in artificial sea water until it is noted that cells are released from marginal portions of the thalli. At the stage where cells have been released from a large number of thalli, the culture medium containing them are centrifuged and the resulting residue is collected. In the case of carpospores, mature thalli of laver are placed in artificial sea water and cultured at a water temperature of 15°C under illumination at a light intensity of 1,500 lux with alternation of 12-hour light periods and 12-hour dark periods until they liberate carpospores. Then, the culture medium containing these carpospores is centrifuged and the resulting residue is separated.

In order to obtain conchospores artificially, mature filaments of the *Conchocelis* phase are cultured in artificial sea water with forced aeration until they liberate conchospore (in 3—4 days). Then, the culture medium containing these conchospores is centrifuged and the resulting residue is collected.

In the practice of the present invention, suspensions are prepared by adding an appropriate amount of sterilized artificial sea water to each of the residues comprising monospores, carpospores or conchospores artificially obtained in the above-described manner. Then, these suspensions are subjected to cell fusion according to the procedure given below.

Procedure for cell fusion

In the case of monospores or carpospores, a suspension prepared in the above-described manner is dropped into a Petri dish or the like and allowed to stand until a sediment is formed on the glass surface. To this sediment is added a 54% polyethylene glycol solution as the fusion inducing substance. After standing for an adequate length of time, a high pH-calcium solution (100 mM $CaCl_2$, pH 10.5) is added thereto and allowed to stand at room temperature. Desirably, the addition of a high pH-calcium solution is repeated twice.

Then, the monospores or carpospores treated as above are cultured in a culture medium comprising artificial sea water. Thus, the formation of homokaryons from monospores or carpospores can be confirmed by observation under the microscope.

In the case of conchospores, a conchospore suspension prepared in the above-described manner is dropped onto a hole slide made of glass. Then, a fusion inducing substance comprising, for example, a 15—20% (w/w) polyethylene glycol solution, high-molecular-weight dextran in sulfuric acid, a highly concentrated sodium nitrate solution, a high pH-high Ca solution (50 mM $CaCl_2$, pH 10.5) or the like is added thereto and allowed to stand at room temperature for 40—50 minutes. Thus, the occurrence of cell membrane fusion as a result of the treatment with a fusion inducing substance such as polyethylene glycol can be confirmed by observation under the microscope.

The occurrence of cell membrane fusion may be confirmed, for example, by reference to the schematic illustration of various stages of cell fusion which is given in Senda et al., "Cell Fusion in Plants" [Cells, 12(8), 1980].

It is to be understood that essentially no fusion of monospores, carpospores or conchospores is noted unless these reproductive cells are treated according to the above-described procedure for cell fusion.

When the homokaryons resulting from the above-described cell fusion of monospores, carpospores or conchospores are cultured in sterilized artificial sea water, more than 90% of them produce a rhizoid to form a cell wall, grow by cell division, and eventually produce thalli of laver.

As described above the present invention enables the cell fusion of laver not by using protoplasts whose preparation is said to involve much difficulty, but by using monospores obtained by culturing young thalli of laver, carpospores obtained by culturing mature thalli of laver, or condhospores liberated from the *Conchocelis* phase of laver.

In addition, the use of such monospores, carpospores or conchospores for the cell fusion of laver has the advantage of eliminating the necessity for a hypertonic solution as used in the cell fusion of protoplasts prepared from terrestrial plants.

More specifically, according to reports on the cell fusion of terrestrial plants, it is generally recognized that protoplasts obtained by treatment with an enzyme such as cellulase will burst unless they are handled in a hypertonic solution. Thus, it is required to handle them in a hypertonic solution containing 0.4—0.7M mannitol or sorbitol.

However, this means that, when the fused protoplasts are to be regenerated, they must be cultured in a hypertonic culture medium. The higher osmotic pressure of the outside of the protoplasts may cause damage to the cell membrane present at the surface of the protoplasts and diable them from growing in the normal state.

On the contrary, monospores, carpospores and conchospores for use in the cell fusion method of the present invention will never burst of themselves even when cultured in a culture medium containing no mannitol or sorbitol, as may be understood from the fact that, under natural conditions, they are liberated from thalli of laver or the Conchocelis phase thereof, float in the sea water and then become attached to laver netting. Thus, these reproductive cells are characterized in that their growth will never encounter the above-described interference.

To further illustrate the present invention, the following examples are given. However, these examples are not to be construed to limit the scope of the invention.

Example 1

Preparation of a monospore suspension

Young thulli of *Porphyra tenera* having a size of 0.2—1.0 mm were placed in sterilized artificial sea water (Asp 12) having the composition given below and cultured at a temperature of 18°C under illumination at a light intensity of 4,000 lux (with alternation of 8-hour light periods and 16-hour dark periods). At the stage where the liberation of monospores was noted, the culture medium was centrifuged at 1,000 rpm for 5 minutes and the resulting residue was collected. Then, a monospore suspension was prepared by adding thereto an appropriate amount of a culture medium comprising the aforesaid artificial sea water.

Composition of artificial
sea water (Asp 12)

| | |
|---|---|
| NaCl | 28 g |
| $MgSO_4 \cdot 7H_2O$ | 7 g |
| $MgCl_2 \cdot 6H_2O$ | 4 g |

| | |
|---|---|
| KCl | 700 mg |
| $CaCl_2 \cdot 2H_2O$ | 1.47 g |
| $NaNO_3$ | 100 mg |
| $K_2HPO_4$ | 10 mg |
| Sodium glycerophosphate | 10 mg |
| Vitamin $B_{12}$ | 0.2 µg |
| Biotin | 1 µg |
| Thiamine | 100 µg |
| P II metal | 10 ml |
| S II metal | 10 ml |
| Trisaminomethane | 1 g |
| Distilled water | 1,000 ml |
| pH | 8.0—8.1 |

Composition of P II metal

| | |
|---|---|
| EDTA | 1 mg |
| $H_3BO_3$ | 1 mg |
| $MnCl_2 \cdot 4H_2O$ | 0.14 mg |
| $FeCl_2 \cdot 6H_2O$ | 0.05 mg |
| $ZnCl_2$ | 0.01 mg |
| $CoCl_2 \cdot 6H_2O$ | 4 µg |
| $CuSO_4 \cdot 5H_2O$ | 0.5 µg |
| Distilled water | 1 ml |

Composition of S II metal

| | |
|---|---|
| NaBr | 1.2 mg |
| $AlCl_3 \cdot 6H_2O$ | 1.2 mg |
| $SrCl_2 \cdot 6H_2O$ | 0.6 mg |
| $NaMoO_4 \cdot 2H_2O$ | 0.12 mg |
| PbCl | 0.03 mg |
| KI | 1.5 µg |
| Distilled water | 1 ml |

Plasmogamy of monospores:

Using a pipette, 0.1 ml of the monospore suspension prepared in the above-described manner was dropped into a Petri dish and allowed to stand for 5—10 minutes until a sediment was formed on the glass surface. Then, 0.2 ml of a polyethylene glycol solution having the composition given below was added to the sediment and allowed to stand for 30 minutes. Thereafter, 0.5 ml of a high pH-calcium solution having the composition given below was added thereto and allowed to stand for 10 minutes. Furthermore, another 0.5 ml of the high pH-calcium solution was added thereto and allowed to stand for 5 minutes.

Composition of polyethylene glycol solution

To a 54% aqueous solution of polyethylene glycol (MW 6,000) were added $CaCl_2 \cdot 2H_2O$, $KH_2PO_4 \cdot H_2O$ and glucose so as to give final concentrations of 10.5 mM, 0.7 mM and 0.1 M, respectively.

Composition of high pH-calcium solution

(1) A solution of 100 mM $CaCl_2 \cdot H_2O$ and 0.4 M glucose in distilled water and (2) a solution of 0.4 M glucose in a NaOH-glycine buffer (pH 10.5) were mixed in a volume ratio of 1:1 just prior to use.

After the above-described treatment, 0.3 ml of a culture medium (artificial sea water Asp 12) was added to the Petri dish, allowed to stand for 5 minutes, and 0.3 ml of the culture medium was sucked out. This procedure was repeated five times and, thereafter, a fresh culture medium (artificial sea water Asp 12) was added to culture the monospores. In the resulting culture medium, the formation of homokaryons from monospores was confirmed.

The formation of homokaryons was also confirmed when thalli of *Porphyra yezoensis* having a size of 1.0—10 cm were used in place of the young thalli of *Porphyra tenera*.

Culture of homokaryons

The homokaryons obtained as above were isolated by sucking them up with an Injectoscope (Olympus Co.), and ·cultured in the aforesaid artificial sea water Asp 12 under the conditions given below.

Culture conditions

| | |
|---|---|
| Temperature | 16—17°C |
| Light intensity | 5,000 lux |
| Light period | 9 hours |
| Dark period | 15 hours |

Four to seven days after commencement of the culture, the formation of a cell wall in the homokaryons was observed and, moreover, several homokaryons exhibited cell division. After the lapse of 3 weeks, they grew to be thalli of a size observable with the naked eye.

Example 2

Preparation of a carpospore suspension

Mature thalli of laver were placed in the same artificial sea water (Asp 12) as used in Example 1 and cultured at a temperature of 15°C under illumination at a light intensity of 1,5000 lux (with alternation of 12-hour light periods and 12-hour dark periods). The liberation of carpospores was noted after 3—4 days of culture. At that time, the culture medium was centrifuged at 1,000 rpm for 5 minutes and the resulting residue was collected. Then, a carpospore suspension was prepared by adding thereto an appropriate amount of the aforesaid artificial sea water Asp 12 as the culture medium.

Plasmogamy of carpospores

Cell fusion was carried out in the same manner as described in Example 1, except that the aforesaid carpospore suspension was used in place of the monospore suspension.

After culturing the carpospores, the culture medium was examined under the microscope to confirm the formation of homokaryons from carpospores.

Culture of homokaryons

The homokaryons obtained as above were cultured in the same manner as described in Example 1. After 3 weeks, they grew to be thalli of a size observable with the naked eye.

Example 3
Preparation of a conchospore suspension

Mature filaments of the *Conchocelis* phase of laver were placed in sterilized artificial sea water (Asp 12) commonly used for the study of algae and having the composition given below, and cultured with forced aeration at a temperature of 17—18°C under illumination at light intensity of 4,000 lux (with alternation of 8-hour light periods and 16-hour dark periods).

The liberation of conchospores was noted after 3—4 days of culture. At that time, the culture medium was centrifuged at 1,000 rpm for 5 minutes and the resulting residue was collected. Then, a conchospore suspension was prepared by adding thereto an appropriate amount of the aforesaid sterilized artificial sea water.

### Composition of artificial sea water (Asp 12)

| | |
|---|---|
| NaCl | 28 g |
| $MgSO_4 \cdot 7H_2O$ | 7 g |
| $MgCl_2 \cdot 6H_2O$ | 4 g |
| KCl | 700 mg |
| $CaCl_2 \cdot 2H_2O$ | 1.47 g |
| $NaNO_3$ | 100 mg |
| $K_2HPO_4$ | 100 mg |
| Sodium glycerophosphate | 10 mg |
| Vitamin $B_{12}$ | 0.2 µg |
| Biotin | 1 µg |
| Thiamine | 100 µg |
| P II metal | 10 ml |
| S II metal | 10 ml |
| Trisaminomethane | 1 g |
| Distilled water | 1,000 ml |
| pH | 8.0—8.1 |

### Composition of P II metal

| | |
|---|---|
| EDTA | 1 mg |
| $H_3BO_3$ | 1 mg |
| $MnCl_2 \cdot 4H_2O$ | 0.14 mg |
| $FeCl_2 \cdot 6H_2O$ | 0.05 mg |
| $ZnCl_2$ | 0.01 mg |
| $CoCl_2 \cdot 6H_2O$ | 4 µg |
| $CuSO_4 \cdot 5H_2O$ | 0.5 µg |
| Distilled water | 1 ml |

### Composition of S II metal

| | |
|---|---|
| NaBr | 1.2 mg |
| $AlCl_3 \cdot 6H_2O$ | 1.2 mg |
| $SrCl_2 \cdot 6H_2O$ | 0.6 mg |
| $NaMoO_4 \cdot 2H_2O$ | 0.12 mg |
| PbCl | 0.03 mg |
| KI | 1.5 µg |
| Distilled water | 1 ml |

Cell fusion of conchospores

150 µl of the conchospore suspension prepared in the above-described manner was dropped onto a hole slide made of glass. Then, 450 µl of a polyethylene glycol solution having the composition given below was slowly added thereto as the fusion inducing substance and allowed to stand at room temperature for 40—50 minutes.

### Composition of polyethylene glycol solution

| | |
|---|---|
| Polyethylene glycol | 45 g |
| $CaCl_2 \cdot 2H_2O$ | 735 mg |
| Distilled water | 100 ml |

After the above-described treatment with polyethylene glycol, 500 µl of the aforesaid sterilized artificial sea water was slowly added thereto and allowed to stand at room temperature for 10 minutes. Then, the hole slide was gently washed with sterilized artificial sea water and placed in a Petri dish having an internal diameter of 9 cm, into which 20 ml of sterilized artificial sea water was gently poured. Thus, the fused conchospores were cultured at a temperature of 18°C under illumination at a light intensity of 7,000 lux (with alternation of 8-hour light periods and 16-hour dark periods). The culture medium was changed every other day.

It was confirmed that they grew to be thalli having a length of the order of 100 microns after 10 days of culture and thalli having a length of the order of 1—1.5 mm after 20 days of culture.

## Claims

1. A method for the cell fusion of laver which comprises subjecting reproductive cells of laver to cell fusion with the aid of a fusion inducing substance, said reproductive cells being selected from the group consisting of monospores, carpospores and conchospores produced in the life cycle of laver.

2. A method as claimed in claim 1 wherein said fusion inducing substance is polyethylene glycol.

3. A method as claimed in claim 1 wherein the cell fusion of monospores is carried out in the presence of a high pH-calcium solution.

4. A method as claimed in claim 1 wherein the cell fusion of carpospores is carried out in the presence of a high pH-calcium solution.

5. A method as claimed in claim 1 wherein said reproductive cells comprise monospores, carpospores or conchospores suspended in artificial sea water.

## Patentansprüche

1. Verfahren zur Zellverschmelzung von See-Lattich (bzw. Purpurtang), dadurch gekennzeichnet, daß man Fortpflanzungszellen von See-Lattich (bzw. Purpurtang) mit Hilfe einer Verschmelzung induzierenden Substanz der Zellverschmelzung unterwirft, wobei die Fortpflanzungszellen ausgewählt werden aus der Gruppe bestehend aus im Lebenszyklus von See-Lattich (bzw. Purpurtang) erzeugten Monosporen, Karposporen und Conchosporen.

2. Verfahren nach Anspruch 1, dadurch gekennzeichnet, daß die Verschmelzung induzierende Substanz Polyethylenglykol ist.

3. Verfahren nach Anspruch 1, dadurch gekennzeichnet, daß die Zellverschmelzung von Monosporen in Gegenwart einer Calciumlösung mit hohem pH-Wert durchgeführt wird.

4. Verfahren nach Anspruch 1, dadurch gekennzeichnet, daß die Zellverschmelzung von Karposporen in Gegenwart einer Calciumlösung mit hohem pH-Wert durchgeführt wird.

5. Verfahren nach Anspruch 1, dadurch gekennzeichnet, daß die Fortpflanzungszellen in künstlichem Seewasser suspendierte Monosporen, Karposporen oder Conchosporen umfassen.

**Revendications**

1. Procédé pour la fusion de cellules de laitue de mer selon lequel on soumet des cellules reproductrices de laitue de mer à une fusion de cellules à l'aide d'une substance induisant la fusion, ces cellules reproductrices étant choisies dans le groupe consistant en monospores, carpostores et conchospores produites dans le cycle de vie de la laitue de mer.

2. Procédé suivant la revendication 1, dans lequel cette substance induisant la fusion est le polyéthylène glycol.

3. Procédé suivant la revendication 1, dans lequel la fusion cellulaire des monospores est effectuée en présence d'une solution de calcium à pH élevé.

4. Procédé suivant la revendication 1, dans lequel la fusion cellulaire des carpospores est effectuée en présence d'une solution de calcium à pH élevé.

5. Procédé suivant la revendication 1, dans lequel ces cellules reproductrices comprennent des monospores, des carpospores ou des conchospores en suspension dans de l'eau de mer artificielle.